# EUROPÄISCHE PATENTSCHRIFT

(11) **EP 1 067 419 B1**
(45) Veröffentlichungstag und Bekanntmachung des Hinweises auf die Patenterteilung: **30.01.2008**
(21) Anmeldenummer: 00112340.5
(22) Anmeldetag: 09.06.2000
(51) Int. Cl.: G02B 7/00, G02B 21/00, A61B 19/00, F16M 11/04

(54) **Deckenstativ**
Ceiling mount
Support fixé au plafond

(30) Priorität: 03.07.1999 CH 122599
(43) Veröffentlichungstag der Anmeldung: 10.01.2001
(73) Patentinhaber: Leica Microsystems AG, 9435 Heerbrugg (CH)
(72) Erfinder: Metelski, Andreas, 8590 Romanshorn (CH)
(74) Vertreter: Rosenich, Paul

(56) Entgegenhaltungen:
- EP-A- 0 257 299
- WO-A-97/20166
- DE-A- 2 061 662
- DE-B- 1 193 897
- FR-A- 2 448 681
- FR-A- 2 759 580
- US-A- 3 566 872
- US-A- 4 035 057
- US-A- 4 531 816

## Beschreibung

Die Erfindung betrifft ein Deckenstativ für ein Mikroskop, insbesondere ein Operationsmikroskop.

Deckenstative sind in verschiedensten Anwendungsgebieten im Einsatz und werden in Abhängigkeit von den unterschiedlichen Anwendungsanforderungen unterschiedlich aufgebaut

Ein großer Anwendungsbereich findet sich in der Intensivmedizin, wo medizinische Geräte, Ablagen, Instrumenten-Halterungen etc. im Bereich des Patienten möglichst flexibel manövrierbar sein sollen.

Von der Firma Dräger sind unter der Produktbezeichnung "Movita, Ondal, Julian, Sola" eine Serie von Deckenstativen erhältlich, die in der Intensivmedizin angewendet werden.

Deckenstative sind aber auch im Bereich der Operationsmikroskopie bekannt und werden vor allem dort eingesetzt, wo der Operationsbereich stationär ist, d.h. wo das Operationsmikroskop den Raum nicht verlässt.

Im Unterschied zu herkömmlichen Bodenstativen, die meist verschiebbar ausgebildet sind, sind Deckenstative häufig an einem Punkt (dem Befestigungspunkt an der Decke - meist eine Deckenkonsole) lagefest. Das Gewicht des gesamten Aufbaus und vorhandene Kippmomente werden an diesem Punkt aufgenommen.

Verschiebbare Bodenstative verfügen demgegenüber häufig über ein Ausgleichsgewicht, das das Gewicht des Mikroskops über einen vertikalen Ständer ausbalanciert, so dass das Stativ nicht kippt. Bei einem älteren Stativ (MS-C) der Anmelderin wurde als Teil des Ausgleichsgewichts ein Apparateschrank mit der Steuerung und der Energieversorgung für das Mikroskop verwendet. Dieser Schrank ist auf dem senkrechten Ständer des Stativs montiert, wobei ein Mechanismus verhindert, das der Schrank und das Mikroskop einseitig in eine Richtung ragen, was zu einem Kippen des Stativs führen kann.

Moderne Mikroskope verfügen über eine entsprechende Steuer- und Energieversorgung, die in einem Apparateschrank untergebracht sind. Daher stellt sich beim Bau eines Deckenstativs die Frage, in welcher Form dieser Apparateschrank montiert werden soll.

Die US-Patentschrift US-3,566,872 offenbart eine Deckenstativkonstruktion, bei der ein Anästhesieapparat auf einem zweiten Tragarm angeordnet ist, der dem Haupttragarm für ein Operationsmikroskop gegenüberliegt. Diese Anordnung geschieht aus Gründen der besseren Bedienbarkeit durch den Chirurgen und zur Vermeidung behindernder Kabel im Operationsraum.

Hierbei ergibt sich ein Schwingungsproblem, da im Falle der Bewegung des Apparateschranks die dabei auftretenden Schwingungen unwillkürlich an das Mikroskop weitergegeben werden. Umgekehrt würden Bewegungen des Mikroskops zu einer Schwingungsanregung des Apparateschranks führen, die dann wieder rückkoppeln könnten.

Das Problem der Schwingungsdämpfung findet sich generell im Stativbau. In dem von der Anmelderin vertriebenen Bodenstativ "OHS" wurde, durch eine besondere Formgebung und Materialwahl der Träger und insbesondere auch durch eine besondere Materialwahl von dämpfenden Abstellfüßen, gegenüber dem Boden eine gute Dämpfungseigenschaft erzielt. Bei einem Deckenstativ verhält sich das Schwingungsverhalten jedoch grundsätzlich anders, da das Deckenstativ gegenüber einem Gebäudeteil starr befestigt ist. Die Lehren, einer Schwingungsrückkopplung durch ungenügend dämpfende Abstellfüße konnten hier grundsätzlich nicht greifen. Dämpfende Abstellfüße können naturgemäß auch nicht vorgesehen werden.

Das Deckenstativ wird mangels einer Dämpfungsmöglichkeit gegenüber einem festen Gebäudeteil in seinem Verlauf mit wenigstens einer dämpfenden Schnittstelle ausgestattet. Diese Schnittstelle ist in sich modular aufgebaut und enthält an sich bekannte dämpfende und nichtdämpfende Schichten. Erst die Kombination aus dämpfenden und nichtdämpfenden Schichten an den Schnittstellen des Stativs ergibt die gewünschten Dämpfungserfolge.

Die Schnittstelle erlaubt grundsätzlich, dass davor und dahinter ein unterschiedlicher Schwingungsvorgang stattfindet, der sich im Idealfall gegenseitig eliminiert. Die Anordnung der Dämpfungsschichten an den horizontalen Schnittstellen der unterteilten horizontalen Träger hat sich als besonders günstig herausgestellt.

Gemäß einer besonderen Ausgestaltung sind die steifen und elastischen Schichten miteinander zu einem Sandwichelement integriert. Dieses lässt sich im Bedarfsfall auch leicht ersetzen bzw. gegen andere Elemente mit unterschiedlichen Dämpfungseigenschaften austauschen.

Es kann von Vorteil sein, wenn an wenigstens einer Schnittstelle zwischen den Tragarmen bzw. den Tragarmteilen federnde oder schwingungsdämpfende Elemente, wie Tellerfedern mit Reibflächen, pneumatische oder hydraulische Dämpfkissen oder Dämpfungszapfen, angeordnet sind.

Anstelle von elastomeren Dämpfungsschichten können im Rahmen der Erfindung auch Tellerfedempakete mit aneinander reibenden Dämpfungsflächen oder pneumatische oder hydraulische Dämpfungspolsterringe mit querschnittsreduzierenden Verbindungsröhren verwendet werden. Dazu könnten auch verschiedene elastomere oder nichtelastomere Dämpfungsschichten oder Segmente miteinander verbunden werden. Sie können z.B. in Bohrungen eines Sandwichelements mit zapfenförmigen Dämpfungselementen angeordnet sein. Daraus kann sich auch eine bessere Flächenpressung ergeben.

Eine besonders gute Schwingungsdämpfung ergibt sich, wenn wenigstens einer der Tragarme als Parallelogramm aufgebaut ist und mit einer diagonal erstreckten Gasdruckfeder abgestützt bzw. gedämpft ist.

Bei einem Aufbau mit einem mehrteiligen Horizontalarm tritt das Problem auf, dass es, durch die beschränkte Steifigkeit eines horizontalen Mikroskoptragarmes, beim Abwinkeln dieses horizontalen Tragarmes um eine vertikale Schwenkachse, aufgrund des Gewichtes der weiteren Tragarme und des Mikroskops, zu einer Torsion im Tragarm, der am vertikalen Träger montiert ist, kommt. Diese Torsion hat ein seitliches Absinken des Mikroskops, relativ zu der Position des Mikroskops bei gestreckten Horizontalarmen, zur Folge. Da der Aktionskreis des Mikroskops um die Abwinkelachse somit auf einer nicht waagrechten Ebene liegt, kommt es, bei guter Lagerung mit geringen Lagerreibungskräften, zwangsläufig zu einem "Aus-der-Position-Laufen" des Mikroskops im nichtgebremsten Zustand, so lange bis das Mikroskop seine tiefste Lage erreicht hat.

Dies ist insofern unangenehm, als der Benutzer von einem Mikroskop Positionstreue verlangt. Gemäß einer naheliegenden Maßnahme könnte man dieses Problem durch das Einfügen von elektrischen Bremsen um die betroffenen Vertikalachsen eindämmen. Dieses würde jedoch einen zusätzlichen materiellen Aufwand bedeuten und außerdem auch das Gewicht des Stativs insgesamt erhöhen.

Das Problem wird dadurch gelöst, dass die Aktionskreisebene in eine wenigstens annähernd horizontale Lage geschwenkt wird. Dabei wird bevorzugt von einer Normalarbeitsstellung des Stativs bzw. Mikroskops ausgegangen, in der es am häufigsten verwendet wird.

Um eine von der Richtung unabhängige Einstellung zu gestatten, muss beim Einstellen des Aktionskreises darauf geachtet werden, dass der absolute Torsionswinkel α des tordierenden horizontalen Mikroskoptragarmes aus der Horizontalen auf beide Seiten des Tragarms gleich ist; d.h. dass der Winkel α, von der Stirnseite des Tragarms gesehen, spiegelsymmetrisch gleich ist, ob der Tragarm nach links oder rechts abgewinkelt ist.

Eine Schrägstellung der vertikalen Drehachse hat zur Folge, dass bei gestreckter Horizontalarmlage das Mikroskop ebenso tief zu liegen kommt, wie bei einer Abwinkelung. Da die Torsionswinkelbildung abhängig vom Gewicht des Mikroskops und seinen Aufbauten ist, wird bevorzugt ein Mittelwert bei mittlerem Arbeitsgewicht gewählt. Dabei wird die Einstellung der Aktionskreisneigung für eine durchschnittliche Mikroskopausrüstung und für die häufigste Mikroskoplage vorgenommen und die Aktionskreisneigungseinstellung anwenderbedienbar ausgebildet.

Die Erfindung wird anhand von Ausführungsbeispielen mit Hilfe der schematischen Zeichnungen näher erläutert. Es zeigen:

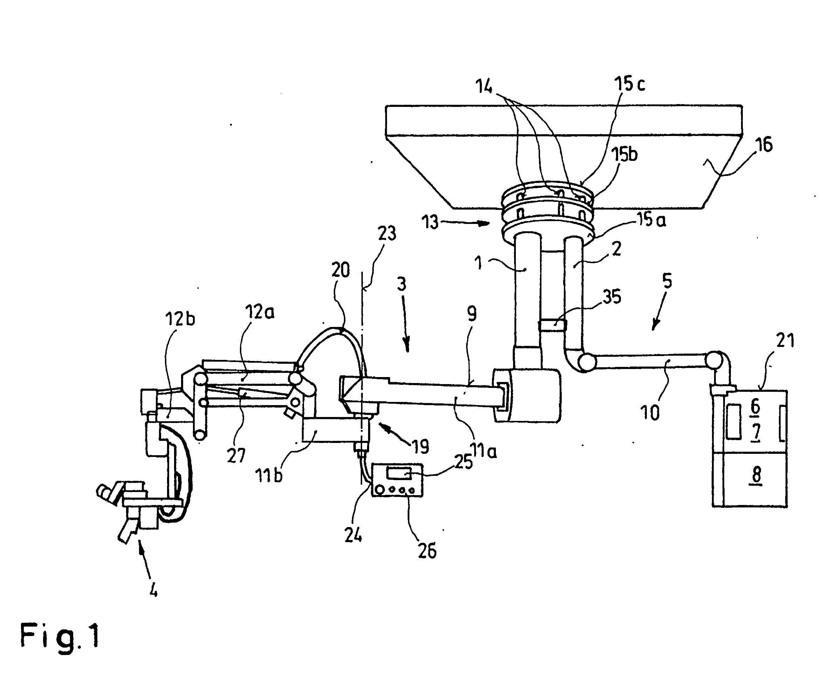

- Fig. 1: ein erfindungsgemäßes Deckenstativ im ausgefahrenen Zustand;

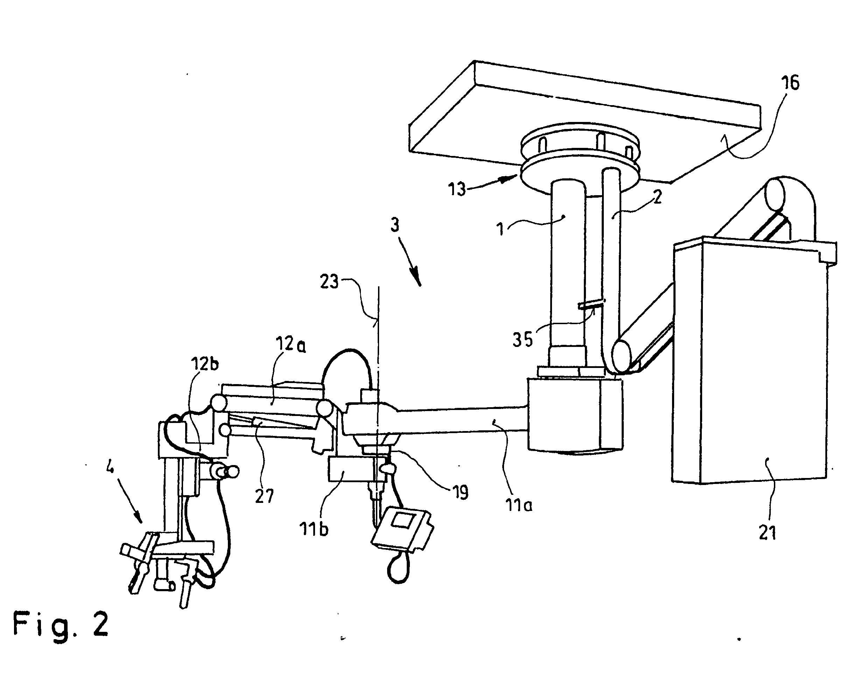

- Fig. 2: das Stativ nach Fig. 1 mit angehobenem und geschwenktem Apparateschrank und etwas geschwenktem Mikroskopstativ;
- Fig. 3: das Stativ nach Fig. 1 in einer Parkposition

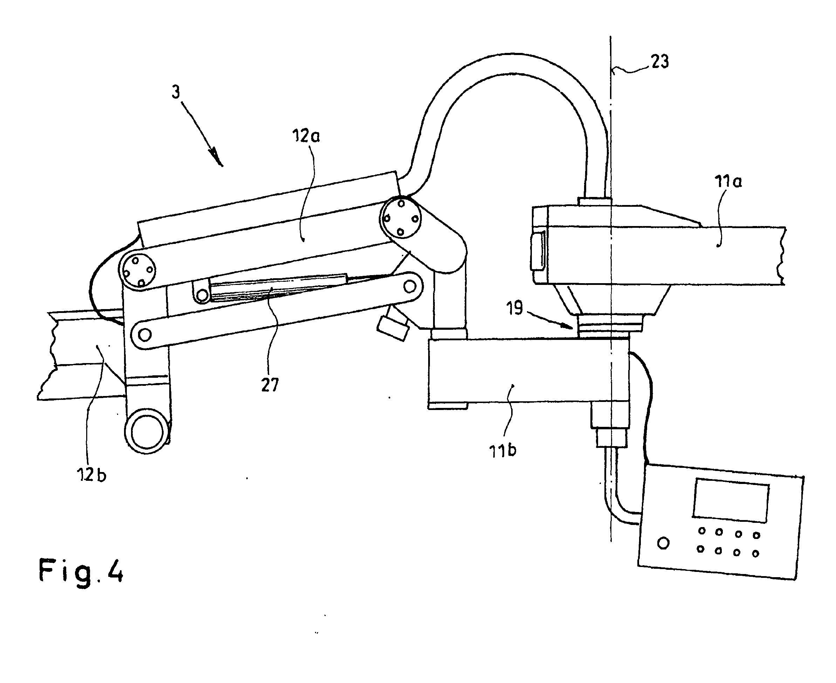

- Fig. 4: ein Detail des Mikroskopstativs;
- Fig. 5: ein vergrößertes Detail mit eingebauter Dämpfungsschnittstelle;
- Fig. 6: ein Schema einer Aktionskreisneigungseinstellung des Mikroskopstativs im Schnitt;
- Fig. 7: eine Draufsicht auf den Aufbau nach Fig. 6;

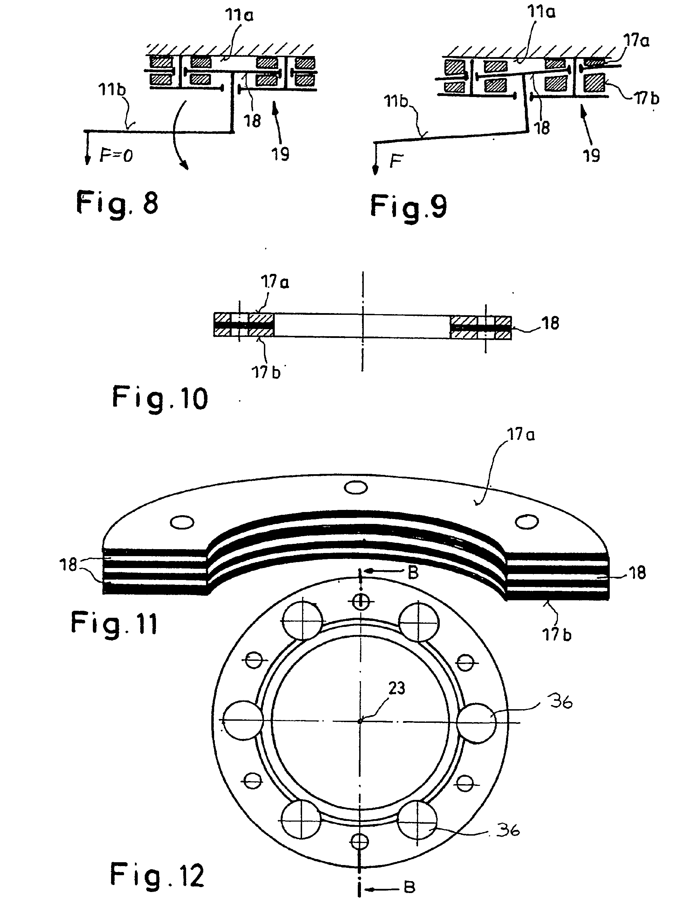

- Fig. 8: ein Schema einer Dämpfungsschnittstelle im Schnitt im unbelasteten Zustand;
- Fig. 9: das Schema nach Fig. 8 im belasteten Zustand;
- Fig. 10: einen Schnitt nach B (Fig. 12) durch ein erfindungsgemäßes Dämpfungselement;
- Fig. 11: eine Hälfte eines anderen erfindungsgemäßen Dämpfungselements in Sandwichbauweise;
- Fig. 12: eine Draufsicht auf das Dämpfungselement von Fig. 10;
- Fig. 13: einen Schnitt A (Fig. 14) durch eine andere Aktionskreisneigungseinstellung des Mikroskopstativs;
- Fig. 14: einen Schnitt B auf die Aktionskreisneigungseinstellung gemäß Fig. 13;
- Fig. 15: ein Schema der Aktionskreisneigungseinstellung der Fig. 13 und 14;

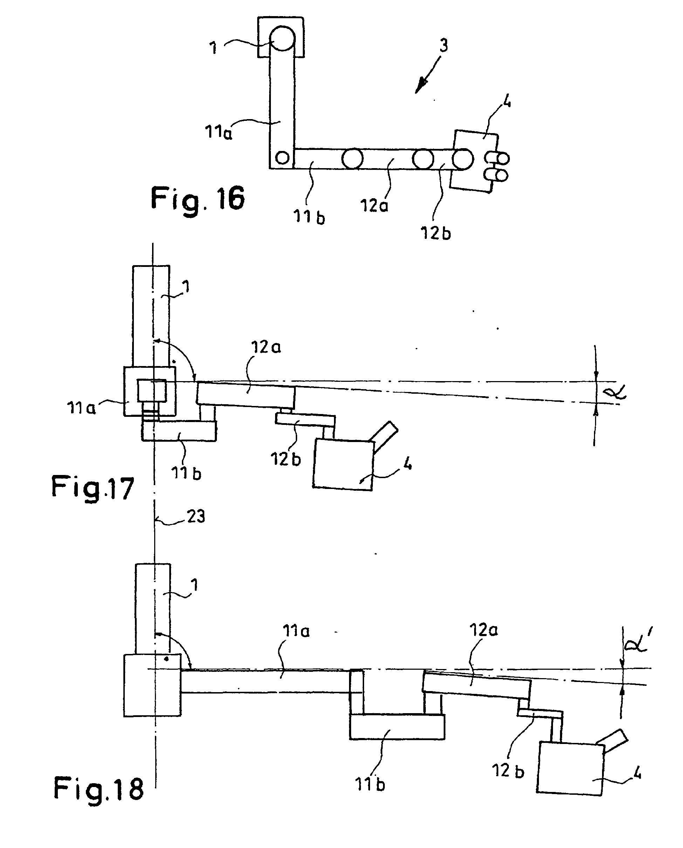

- Fig. 16: die Draufsicht auf ein schematisches Mikroskopstativ in nach rechts abgewinkeltem Zustand;
- Fig.17: die Seitenansicht auf das Mikroskopstativ in der Stellung nach Fig. 16;
- Fig.18: die um 90° gedrehte Seitenansicht auf dasselbe Mikroskopstativ mit gestreckten Tragarmen 11 und 12 und

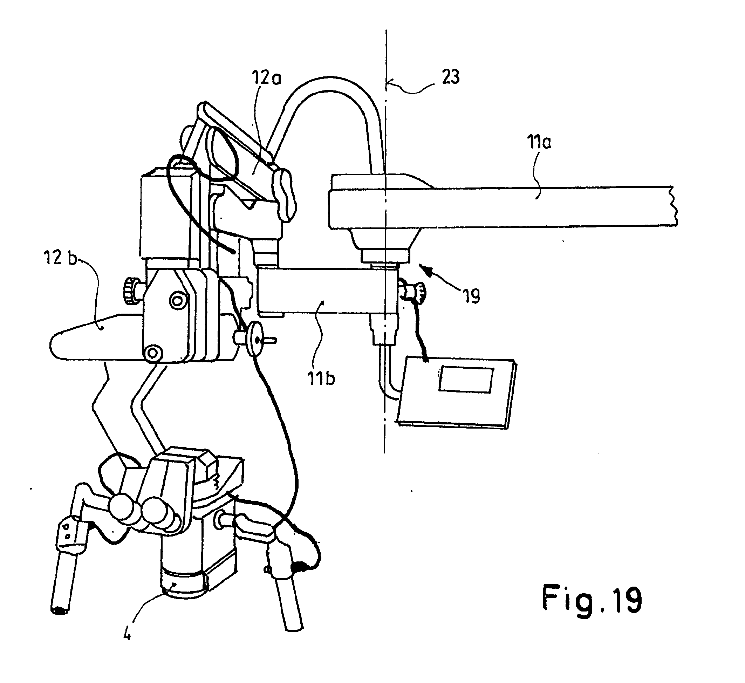

- Fig. 19: ein Stativ nach Fig. 1 in der bevorzugten Arbeitsstellung mit dreimal abgewinkelten Tragarmen 11a, 11b, und 12 a, 12b.

Die Figuren werden übergreifend beschrieben. Gleiche Bezugszeichen bedeuten gleiche oder gleichartige Bauteile mit gleichen Funktionen. Bezugszeichen mit Indizes gehören zu Teilen, die aus mehreren Elementen zusammengesetzt sind.

Die gezeigte Variante eines Deckenstativs verfügt über zwei senkrechte Träger 1, 2, die voneinander unabhängig an einer Deckenkonsole 13 montiert sind. Die Deckenkonsole 13 ist selbst an der Decke 16 eines festen Gebäudeteils befestigt. Der eine Träger 1 trägt das eigentliche Mikroskopstativ 3 mit einem Operationsmikroskop 4. Der andere Träger 2 trägt ein Hilfsstativ 5 mit einem Apparateschrank 21, der bei richtiger Positionierung als Ausgleichsgewicht dient und beispielsweise einen Rechner 6, Steuerungen 7 und Energiequellen 8 und Energiewandlern beinhaltet. Unter Energiequellen sind Lichtquellen zu verstehen, die über ein Glasfaserkabel 20 Licht zum Mikroskop 4 senden. Das Glasfaserkabel 20 wird zusammen mit allen anderen Steuerleitungen vom Apparateschrank 21 über eine Brücke 35, zwischen den beiden Trägern 1 und 2, vom Hilfsstativ 5 zum Mikroskopstativ 3 geführt.

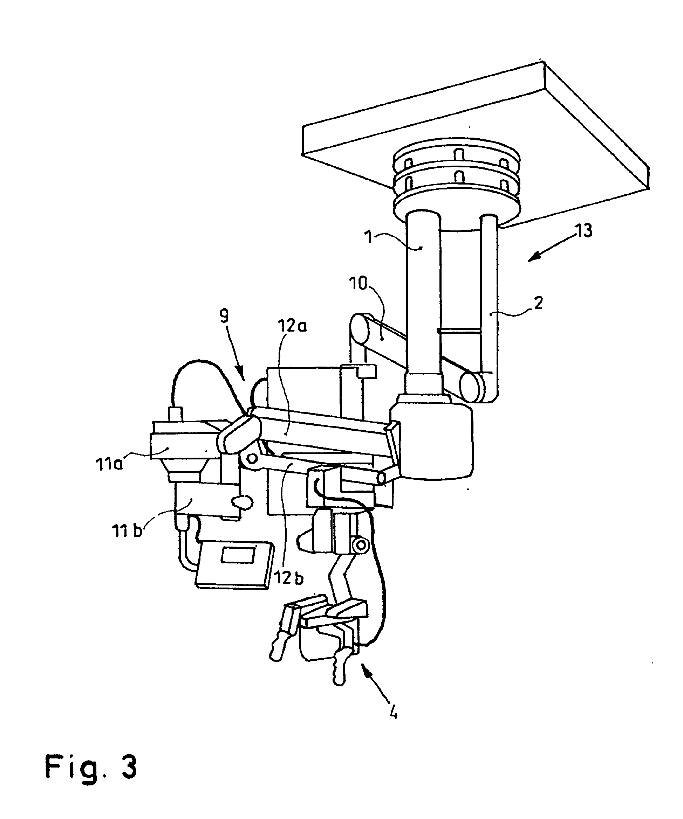

Sowohl das Mikroskopstativ 3, als auch das Hilfsstativ 5 umfassen einen horizontal orientierten Tragarm 9 bzw. einen Ausgleichsarm 10. Der Tragarm 9 für das Operationsmikroskop 4 ist in wenigstens zwei zueinander gelenkig verbundene Tragarmteile unterteilt, von denen jeder nochmals unterteilt ist in 11 a und 11 b bzw. 12a und 12b, während der Ausgleichsarm 10 das Ausgleichsgewicht bzw. den Apparateschrank 21 aufnimmt und in der Regel in geeignetem Abstand vom Operationsmikroskop 4 hält. Lediglich bei Bedarf kann der gesamte Aufbau auch so gefaltet werden, dass Mikroskop 4 und Apparateschrank 21 unmittelbar zueinander in eine Parkposition kommen (Fig. 3). Das kann z.B. eine Versorgungsstellung sein, oder im Bedarfsfall eine Stellung, in der der Anwender beim Blick durch das Mikroskop gleichzeitig auch Manipulationen am Apparateschrank vornehmen möchte. In diesen seltenen Fällen, wird auf die Balancefunktion der beiden Stativteile (Mikroskopstativ bzw. Hilfsstativ) verzichtet und die Last dementsprechend auch asymmetrisch von der Deckenkonsole 13 aufgenommen.

Der Vorteil bzw. Effekt dieses Aufbaus ergibt sich in der Regel u.a. in einer grundsätzlich geringen asymmetrischen Belastung der Deckenkonsole 13, die im Wesentlichen aus mehreren senkrechten Tragsäulen 14 und wenigstens einer waagrechten Tragplatte 15 (im vorliegenden Fall drei Tragplatten 15) besteht, die die senkrechten Träger 1,2 aufnimmt.

In der Regel sind der von den Trägern 1, 2 abragende waagrechte Tragarm 9 und der Ausgleichsarm 10 nämlich einander gegenübergestellt, so dass die jeweils zugehörigen Lasten an einer gedachten Vertikalen aus der Mitte der Deckenkonsole 13 symmetrisch hängen. Dies entlastet einerseits die Deckenmontageeinrichtung 13, dient jedoch auch einer Schwingungsentkopplung und einer Trennung der unterschiedlichen Massen.

Insbesondere kann der Apparateschrank 21 lageverändert werden, ohne dass sich die zwangsläufig damit verbundenen Schwingungen bis zum Operationsmikroskop 4 übertragen.

Das Problem, das durch die Erfindung gelöst wird, ist das zwangsläufige Aus-der Position-Laufen eines Gewichts an den zwei Tragarmteilen 11a, 11 b und 12a, 12b die zueinander um wenigstens eine senkrechte Achse 23 schwenkbar sind, wobei der am senkrechten Träger 1 gehaltene erste Teil des Tragarmteils 11a, um den vertikalen Träger 1 schwenkbar ist. Das Problem ergibt sich aus der endlichen Steifigkeit von Tragarmen. Diese haben nämlich die Tendenz, sich unter der Last des Operationsmikroskops 4 etwas zu verbiegen und zu tordieren. Durch diese Verbiegung sinkt der Schwergewichtspunkt des Mikroskops 4 etwas tiefer, als er nach mathematischen Grundsätzen optimaler Träger sein dürfte. Dadurch entsteht jedoch beim Abwinkeln eines der beiden Tragarmteile11b gegenüber dem anderen 11a um die Achse 23 ein Gewichtsmoment, das zu einem "Verlaufen" - d.h. zu einem Abschwenken des Operationsmikroskops 4 zu seinem tiefsten Punkt entlang seines Aktionskreises - führen kann.

Dieses "Verlaufen" wird dadurch verhindert, dass die vertikale Achse 23 erfingdungsgemäß aus der Vertikalen geneigt angeordnet wird. Die Neigung ergibt sich dabei aus den mechanischen Biegeeigenschaften des Mikroskopaufbaus 3. Dies führt, je nach Abwinkelung der beiden benachbarten Tragarmteilen 11a, 11 b, 12a, 12b zueinander, zu einem "In-die-Waagrechte-Stellen" des vom inneren Tragarmteils 11 a abragenden äußeren Tragarmteils 11b bzw. 12a, 12b. Dabei wird der Schwerpunkt der Last in seiner potentiell richtigen (gleiche Höhenlage) Position gehalten. Seitlich wirkende Kraftkomponenten können erst gar nicht auftreten. Der Aktionskreis des Mikroskops wird somit in eine waagrechte bzw. horizontale Ebene gelegt.

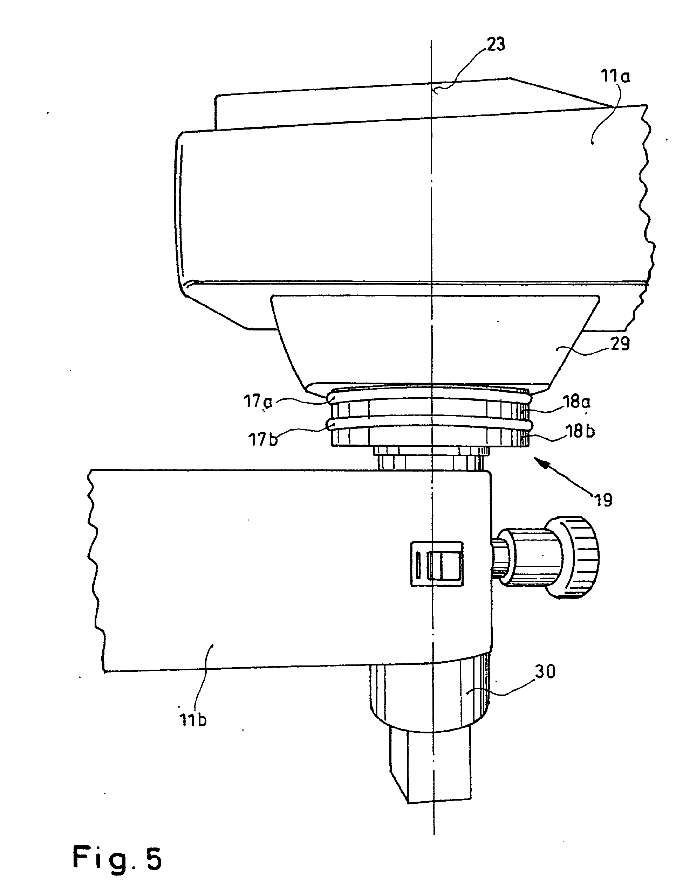

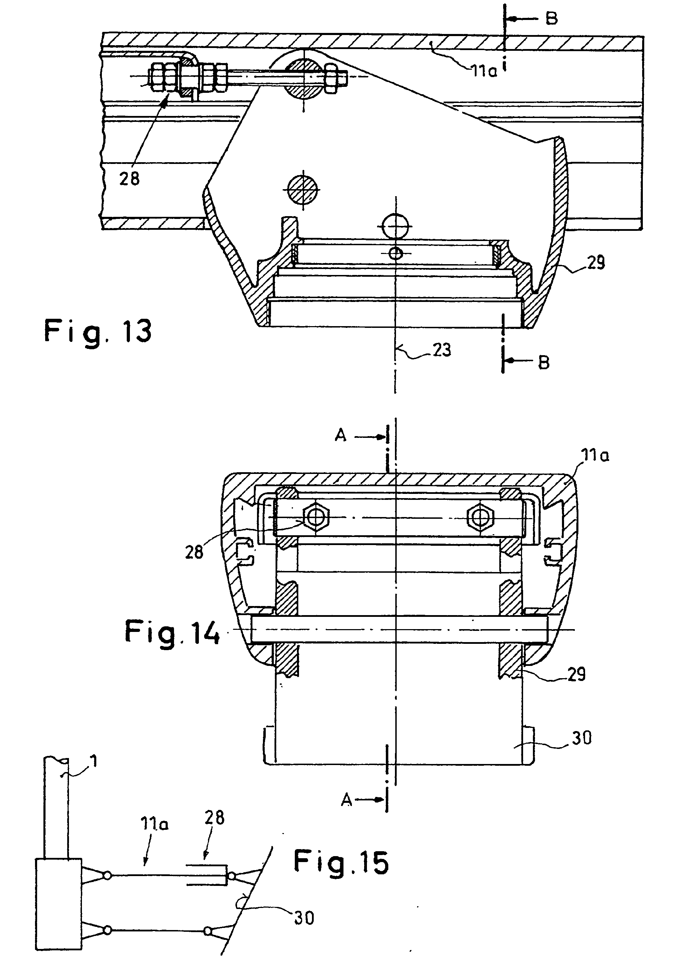

Der bevorzugte Aufbau für das "In die Waage Stellen" des Aktionskreises ist in Fig. 5 und den Fig. 13 bis 15 im Detail dargestellt. Der Tragarmteil 11a nimmt dabei einen Schwenkteil 29 auf, der die Achse 23 hält. Über eine Einstellung 28 (Fig. 13) mit einer Zugstange und Schraubenmuttern ist der Schwenkteil 29 relativ zum Tragarmteil 11a verschwenkbar. Die Achse 23 ist somit leicht um die Vertikale einstellbar. Da das Schwenkteil 29 im montierten Zustand eine Achse 30 trägt (Fig. 14, Fig. 15), an der der zweite Tragarmteil 11b gelagert ist, kann dessen Neigung eingestellt werden. Damit stellt sich auch die Lage des Aktionskreises relativ zur Waagrechten, bzw. Horizontalen ein.

Alternativ zu einer Einstellung 28 kann der Winkel zur Vertikalen einer Achse von vornherein schräg eingestellt sein.

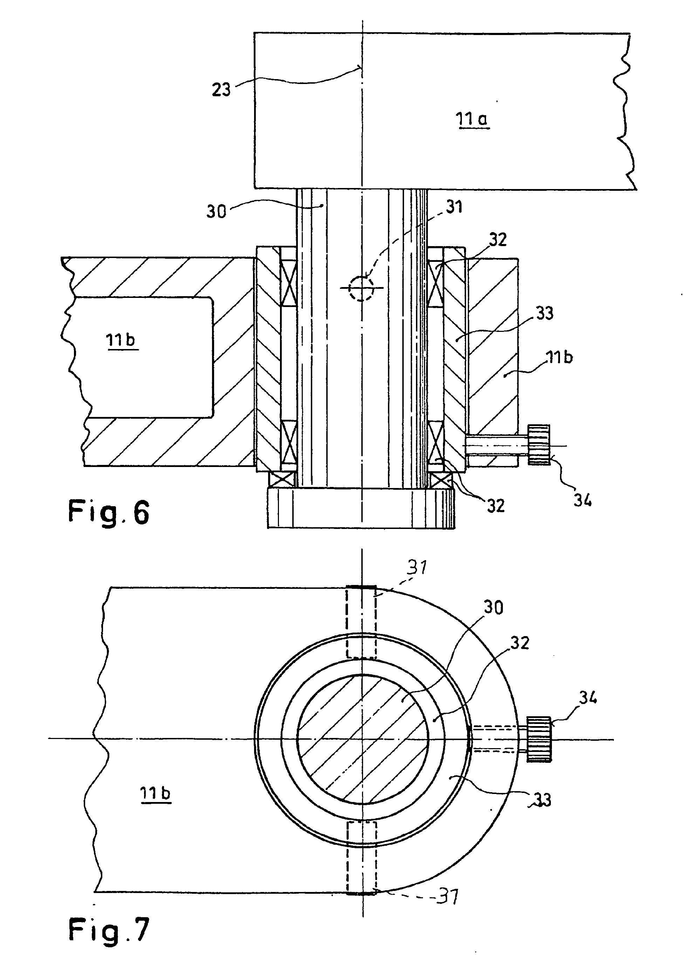

Eine Variante zu diesem Aufbau ist symbolisch in den Fig. 6 und 7 dargestellt.

Die Achse 30 ist bei dieser Variante starr mit dem Tragarmtell 11a verbunden. Sie trägt eine Lagerhülse 33, die gegenüber der Achse 30 mittels Lagern 32 gut drehbar gelagert ist. Die Hülse 33 ist gegenüber dem Tragarmteile 11b an einer kardanischen Aufhängung 31 schwenkbar gelagert. Dazu ist die Hülse 33 im Tragarmteil befestigt. Eine Verstellschraube 34 ermöglicht die Neigungseinstellung der Hülse 33 relativ zum Tragarmteil 11b und damit die relative Neigungseinstellung der Achse 23 um eine Vertikale.

### Bezugszeichenliste

- 1: vertikaler Träger für Mikroskopstativ
- 2: vertikaler Träger für Hilfsstativ
- 3: Mikroskopstativ
- 4: Operationsmikroskop
- 5: Hilfsstativ
- 6: Rechner
- 7: Steuerung
- 8: Energiequelle
- 9: Tragarm
- 10: Ausgleichsarm
- 11: Tragarmteil a, b
- 12: distaler Tragarmteil a, b
- 13: Deckenkonsole
- 14: Tragsäule
- 15: Tragplatte
- 16: fester Gebäudeteil, Decke
- 17: dämpfende Schicht a, b, c
- 18: nicht dämpfende Schicht a, b, c
- 19: dämpfende Schnittstelle, Dämpfungselement
- 20: Glasfaserkabel
- 21: Apparateschrank
- 22: Brücke
- 23: senkrechte Achse
- 24: Konsole
- 25: Display
- 26: Bedienelement
- 27: Gasdruckfeder
- 28: Einstellmittel
- 29: Schwenkteil
- 30: Tragachse
- 31: Kardanische Aufhängung
- 32: Lager
- 33: Lagerhülse
- 34: Verstellschraube
- 35: Brücke
- 36: Dämpfungszapfen

## Patentansprüche

1. Deckenstativ für Mikroskope (4), insbesondere für Operationsmikroskope, mit einer Deckenkonsole (13), einem ersten vertikalen Träger (1) und einem Mikroskopstativ (3), das wenigstens einen horizontalen Tragarm (9) aufweist, der angepasst ist, um ein Mikroskop (4) zu tragen, wobei parallel zum ersten vertikalen Träger (1) ein zweiter vertikaler Träger (2) mit der Deckenkonsole (13) verbunden ist, der ein Hilfsstativ (5) zur Aufnahme eines Ausgleichsgewichtes, insbesondere eines als Ausgleichsgewicht wirkenden Apparateschranks (21) trägt, wobei der Tragarm (9) in wenigstens zwei Tragarmteile (11 a und 11 b) unterteilt ist, die zueinander um wenigstens eine senkrechte Achse (23) schwenkbar sind, so dass das Mikroskop (4) um die Achse (23) einen Aktionskreis beschreibt, und dass die Tragarmteile (11 a, b) wenigstens eine Schnittstelle (19) zueinander aufweisen, **dadurch gekennzeichnet, dass** die Schnittstelle (19) dämpfend ausgerüstet ist, wobei pro Schnittstelle (19) wenigstens zwei Lagen dämpfender Schichten (17 a, b, c) durch wenigstens eine nicht dämpfende Schicht (18 a, b, c) getrennt sind und dass das Deckenstativ mit Mitteln (28-30;31-34) ausgestattet ist, die ein Einstellen der Aktionskreisneigung erlauben, so dass der Aktionskreis in einer wenigstens annähernd horizontalen Ebene liegt, bzw. dass die potentielle Energie der Last in allen Winkellagen annähernd gleich ist, wobei die Mittel eine Neigungsverstellung (28) für die Achse (23) und/oder eine Neigungsverstellung (34) für eine kardanisch aufgehängte Lagerhülse (33) umfassen.

2. Deckenstativ nach Anspruch 1, **dadurch gekennzeichnet, dass** die dämpfende Schnittstelle (19) annähernd horizontal ist.

3. Deckenstativ nach einem der vorhergehenden Ansprüche, **dadurch gekennzeichnet, dass** es an der Schnittstelle (19) wenigstens eine Lage eines elastomeren Dämpfungsmaterials vorweist, das bei einer Pressung von 0-2,5 N/mm² eine Einfederung von max. 20% der Materialdicke aufweist.

## Claims

1. Ceiling mount for microscopes (4), in particular for surgical microscopes, having a ceiling console (13), a first vertical support (1) and a microscope mount (3) which has at least one horizontal support arm (9) which is adapted for supporting a microscope (4), there being connected to the ceiling console (13) in a fashion parallel to the first vertical support (1) a second vertical support (2), which supports an auxiliary mount (5) for holding a balancing weight, in particular an equipment cabinet (21) acting as balancing weight, the support arm (9) being subdivided into at least two support arm parts (11a and 11b) which can be pivoted relative to one another about at least one vertical axis (23) such that the microscope (4) describes an action circle about the axis (23), and that the support arm parts (11a, b) have at least one interface (19) with one another, **characterized in that** the interface (19) is fitted in a damping fashion, at least two plies of damping layers (17a, b, c) being separated per interface (19) by at least one non-damping layer (18a, b, c), and **in that** the ceiling mount is equipped with means (28-30; 31-34) which permit the inclination of the action circle to be set such that the action circle lies in at least an approximately horizontal plane, or that the potential energy of the load is approximately equal in all angular positions, the means comprising an inclination adjuster (28) for the axis (23) and/or an inclination adjuster (34) for a cardanically suspended bearing sleeve (33).

2. Ceiling mount according to Claim 1, **characterized in that** the damping interface (19) is approximately horizontal.

3. Ceiling mount according to one of the preceding claims, **characterized in that** it exhibits at the interface (19) at least one ply of an elastomeric damping material which has a spring deflection of at most 20% of the material thickness under a pressure of 0-2.5 N/mm².

## Revendications

1. Support fixé au plafond pour microscope (4), notamment pour microscope chirurgical, comprenant une console de plafond (13), un premier montant vertical (1) et un support de microscope (3) qui présente au moins un bras de support horizontal (9) qui est conçu pour porter un microscope (4), un deuxième montant vertical (2) étant connecté à la console de plafond (13) parallèlement au premier montant vertical (1), lequel porte un support auxiliaire (5) pour recevoir un contrepoids, notamment une armoire à instruments (21) agissant en tant que contrepoids, le bras de support (9) étant divisé en au moins deux parties de bras de support (11a et 11b) qui peuvent pivoter l'une par rapport à l'autre autour d'au moins un axe vertical (23), de sorte que le microscope (4) décrive autour de l'axe (23) un cercle d'action et que les parties de bras de support (11a,b) présentent au moins une interface (19) l'une par rapport à l'autre, **caractérisé en ce que** l'interface (19) est pourvue d'un amortissement, au moins deux couches de revêtement amortissant (17a,b,c) pour chaque interface (19) étant séparées par au moins un revêtement non amortissant (18a,b,c) et **en ce que** le support fixé au plafond est muni de moyens (26-30 ; 31-34) qui permettent un ajustement de l'inclinaison du cercle d'action de sorte que le cercle d'action se situe dans un plan au moins approximativement horizontal, ou que l'énergie potentielle de la charge soit approximativement égale dans toutes les positions angulaires, les moyens comprenant un réglage de l'inclinaison (28) pour l'axe (23) et/ou un réglage de l'inclinaison (34) pour un manchon de palier (33) à suspension à cardan.

2. Support fixé au plafond selon la revendication 1, **caractérisé en ce que** l'interface à amortissement (19) est approximativement horizontale.

3. Support fixé au plafond selon l'une quelconque des revendications précédentes, **caractérisé en ce qu'**il est prévu au niveau de l'interface (19) au moins une couche de matériau d'amortissement élastomère, qui présente, sous une pression de 0-2,5 N/mm², une déformation élastique de 20 % max. de l'épaisseur de matériau.
